(19)

Europäisches
Patentamt
European
Patent Office
Office européen
des brevets

(11) **EP 3 790 995 B1**

(12) **EUROPEAN PATENT SPECIFICATION**

(45) Date of publication and mention
of the grant of the patent:
**29.04.2026 Bulletin 2026/18**

(21) Application number: **19723089.9**

(22) Date of filing: **08.05.2019**

(51) International Patent Classification (IPC):
***C12Q 1/6886*** *(2018.01)*

(52) Cooperative Patent Classification (CPC):
**C12Q 1/6886;** C12Q 2600/112; C12Q 2600/118;
C12Q 2600/156

(86) International application number:
**PCT/EP2019/061810**

(87) International publication number:
**WO 2019/215223 (14.11.2019 Gazette 2019/46)**

(54) **METHOD OF CANCER PROGNOSIS BY ASSESSING TUMOR VARIANT DIVERSITY BY MEANS OF ESTABLISHING DIVERSITY INDICES**

**VERFAHREN ZUR PROGNOSE VON KREBS DURCH BEURTEILUNG DER TUMORVARIANTENDIVERSITÄT DURCH FESTLEGUNG VON DIVERSITÄTSINDIZES**

**PROCÉDÉ DE PRONOSTIC DU CANCER PAR ÉVALUATION DE DIVERSITÉ DE VARIANTS DE TUMEUR AU MOYEN D'ÉTABLISSEMENT D'INDICES DE DIVERSITÉ**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **08.05.2018 US 201862668684 P**

(43) Date of publication of application:
**17.03.2021 Bulletin 2021/11**

(73) Proprietors:
• **F. Hoffmann-La Roche AG**
**4070 Basel (CH)**
Designated Contracting States:
**AL AT BE BG CH CY CZ DK EE ES FI FR GB GR HR HU IE IS IT LI LT LU LV MC MK MT NL NO PL PT RO RS SE SI SK SM TR**
• **Roche Diagnostics GmbH**
**68305 Mannheim (DE)**
Designated Contracting States:
**DE**

(72) Inventors:
• **MA, Xiaoju**
**Pleasanton, California 94588 (US)**
• **WEHNL, Birgit**
**82377 Penzberg (DE)**
• **YAUNG, Stephanie J.**
**Pleasanton, California 94588 (US)**
• **MULEY, Thomas**
**69126 Heidelberg (DE)**
• **HERTH, Felix**
**69126 Heidelberg (DE)**
• **BALASUBRAMANYAM, Aarthi**
**Pleasanton, California 94588 (US)**
• **XI, Liu**
**Pleasanton, California 94588 (US)**
• **JU, Christine**
**Pleasanton, California 84588 (US)**

(74) Representative: **Hildebrandt, Martin K. E.**
**Roche Diagnostics GmbH**
**Nonnenwald 2**
**82377 Penzberg (DE)**

(56) References cited:
**US-A1- 2017 091 527**

**(Cont. next page)**

- DAGOGO-JACK IBIAYI ET AL: "Tumour heterogeneity and resistance to cancer therapies", NATURE REVIEWS CLINICAL ONCOLOGY, vol. 15, no. 2, 8 November 2017 (2017-11-08), NY, US, pages 81 - 94, XP93043336, ISSN: 1759-4774, Retrieved from the Internet <URL:http://www.nature.com/articles/nrclinonc.2017.166> DOI: 10.1038/nrclinonc.2017.166
- J JIANG ET AL: "Longitudinal Mutation Monitoring in Plasma by Deep Sequencing as a Potential Predictor of Disease Progression in NSCLC", JOURNAL OF THORACIC ONCOLOGY, vol. 12, no. 11S2, 1 November 2017 (2017-11-01), pages S1772 - S1773, XP055611254, DOI: https://doi.org/10.1016/j.jtho.2017.09.386
- CHRISTOPHER ABBOSH ET AL: "Phylogenetic ctDNA analysis depicts early-stage lung cancer evolution", NATURE, vol. 545, no. 7655, 26 April 2017 (2017-04-26), London, pages 446 - 451, XP055409582, ISSN: 0028-0836, DOI: 10.1038/nature22364
- CHUNG YUL RI ET AL: "Diversity index as a novel prognostic factor in breast cancer.", ONCOTARGET 14 NOV 2017, vol. 8, no. 57, 14 November 2017 (2017-11-14), pages 97114 - 97126, XP002793566, ISSN: 1949-2553
- ALEGRE E ET AL: "Total and mutated EGFR quantification in cell-free DNA from non-small cell lung cancer patients detects tumor heterogeneity and presents prognostic value", TUMOR BIOLOGY, KARGER, BASEL, CH, vol. 37, no. 10, 29 July 2016 (2016-07-29), pages 13687 - 13694, XP036092751, ISSN: 1010-4283, [retrieved on 20160729], DOI: 10.1007/S13277-016-5282-9
- ALMENDRO V ET AL: "Inference of tumor evolution during chemotherapy by computational modeling and in situ analysis of genetic and phenotypic cellular diversity", CELL REPORTS, ELSEVIER INC, US, vol. 6, no. 3, 13 February 2014 (2014-02-13), pages 514 - 527, XP002743200, ISSN: 2211-1247, [retrieved on 20140123], DOI: 10.1016/J.CELREP.2013.12.041
- HUI LIU ET AL: "A system for tumor heterogeneity evaluation and diagnosis based on tumor markers measured routinely in the laboratory", CLINICAL BIOCHEMISTRY, ELSEVIER INC, US, CA, vol. 48, no. 18, 26 July 2015 (2015-07-26), pages 1241 - 1245, XP029330623, ISSN: 0009-9120, DOI: 10.1016/J.CLINBIOCHEM.2015.07.027
- PENG MUYUN ET AL: "Non-blood circulating tumor DNA detection in cancer", ONCOTARGET, vol. 8, no. 40, 15 September 2017 (2017-09-15), pages 69162 - 69173, XP002793567

**Description**

**FIELD OF THE INVENTION**

The invention relates to the field of oncology. More specifically, the invention relates to the field of nucleic acid-based testing of cancer patients.

**BACKGROUND OF THE INVENTION**

Many cancer patients are diagnosed at metastatic stage or progress to metastasis from earlier stage disease. At that time, prognosis is poor and the optimal choice of effective therapy is critical. Modern diagnostic approaches rely on mutations found in circulating tumor DNA (ctDNA) to predict tumor resistance and recurrence (*see* U.S. Patent applications Ser. No. 14/774,518 and International app. No. PCT/US2015/049838 titled "Identification and Use of Circulating Tumor Markers"). For example, increasing mutant allele frequencies (AF) of resistance mutations indicate developing resistance to a particular targeted therapy. There is however a need for a more general assessment of tumor evolution from pre- to post-treatment in order to select the appropriate therapy. Abbosh et al., 2017, Nature 545(7655):446-451 used a tumour-specific phylogenetic approach to profile the ctDNA of study participants, and identified independent predictors of ctDNA release and analyse the tumour-volume detection limit. Chung et al., 2017, Oncotarget 8(57):97114-97126 investigated the significance of the Shannon diversity index of gene copy number variation as a tool for measuring genetic heterogeneity in breast cancer.

Mutation data from profiling circulating tumor DNA may contain information that cannot be easily interpreted with the state of the art tools. Each patient at a given time has a collection of circulating tumor variants from primary and metastatic tumors that have shed DNA into the circulation. The presence or absence of specific mutations or mutation burden can be detected with state of the art techniques but not easily interpreted for use in patient care. There is a need to interpret and translate this mutation data into clinically useful information.

**SUMMARY OF THE INVENTION**

The invention is a method of identifying a prognosis for a cancer patient comprising the steps of: (a) isolating nucleic acids from a cell-free blood sample obtained from the patient; (b) determining in the samples the sequence of at least a portion of each of the biomarkers listed in Table 1; wherein said portion may include single-nucleotide variations (SNVs), deletions and insertions (in-dels) that correspond to nonsense missense and frame-shift mutations if they occur in the coding regions of genes, gene fusions or translocations. (c) determining a tumor variant diversity index in the patient; (d) identifying the patient as having a good prognosis if the tumor variant diversity index is in the same quantile as the tumor variant diversity index of patients in a relevant population who have had a good outcome; or (e) identifying the patient as having a poor prognosis if tumor variant diversity is in the same quantile as the tumor variant diversity of patients in the relevant population who have had a poor outcome, wherein the cancer is selected from among non-small cell lung cancer (NSCLC), small cell lung cancer (SCLC), wherein the tumor variant diversity is selected from Shannon diversity index, Simpson diversity index, Inverse Simpson diversity index and Gini-Simpson diversity index, and wherein the prognosis is overall survival (OS).

In some embodiments, the diversity index is determined using the proportion of species in a population determined according to Formula I. The Shannon diversity index can be determined according to Formula II. The Simpson diversity index can be determined according to Formula III. The Inverse Simpson diversity index can be determined according to Formula IV. The Gini-Simpson diversity index can be determined according to Formula V.

In some embodiments, the relevant population is the population of patients having the same type of cancer.

The step of determining the sequence may comprise one or more of target enrichment, adaptor ligation, molecular barcodes, sequence alignment, error correction and DNA amplification.

In another embodiment, the method can be carried out with the help of a computer system designed to detect tumor variant diversity in a patient comprising a processor and a non-transitory computer readable medium coupled to the processor, the medium comprising code executable by the processor for performing a method comprising the steps of analyzing sequencing data on biomarkers from Table 1, performing sequence comparison and mutation detection, error correction, determining tumor variant diversity index according to one or more of Formula II, Formula III, Formula IV and Formula V and whether the tumor variant diversity in the sample falls above or below a predetermined threshold.

**BRIEF DESCRIPTION OF THE DRAWINGS**

**Figure 1** illustrates a relationship between overall survival (OS) and Shannon diversity index in Stage IV small cell lung cancer (SCLC) patients.

**Figure 2** illustrates a relationship between overall survival (OS) and Gini-Simpson diversity index in Stage IV small cell lung cancer (SCLC) patients.

**Figure 3** illustrates a relationship between overall survival (OS) and Gini-Simpson diversity index in Stage IV lung adenocarcinoma (non-small cell lung cancer, NSCLC) patients.

**DETAILED DESCRIPTION OF THE INVENITON**

*Definitions*

The following definitions are not limiting but merely aid in understanding this disclosure.

The term "PFS" is used herein to describe the time of Progression Free Survival for a patient.

The term "OS" is used herein to describe the time of Overall Survival for a patient.

The term "circulating tumor DNA (ctDNA)" is used herein to describe a portion of cell-free DNA (cfDNA) found in human blood plasma or serum that originates from the tumor. Circulating tumor DNA is distinguished from non-tumor DNA by the mutations characteristic of the tumor.

The term "biomarker" is used herein to describe a nucleotide sequence that contains information relevant to the biological or clinical phenomenon. For example, the information may be a mutation status of the nucleotide sequence. The biomarker can be a gene (including coding sequence, regulatory sequence, intron or a splice site) or an intergenic region. The clinical phenomenon can be the presence of malignant cells, e.g., tumor cells in a patient's sample.

The term "diversity index" is used herein to describe a quantitative measure that reflects how many different species there are in a dataset, and simultaneously takes into account how evenly the basic entities are distributed among those species. *See* Magurran, A.E., Measuring Biological Diversity, 2003 Wiley-Blackwell. The terms "tumor diversity index" and "tumor variant diversity index" are used interchangeably to refer to a diversity index applied to mutant sequence variants (species) found in the tumor.

The invention is a method of assessing prognosis of a tumor patient as defined by the claims based on the mutation content of the patient's circulating tumor DNA (ctDNA). Specifically, the mutation content is assessed to determine diversity of tumor cells in the patient.

Results from profiling circulating tumor DNA may contain information beyond what can be assessed by state-of-the-art tools. Each patient at a given time has a collection of circulating tumor variants that contain both primary and metastatic tumors that have shed DNA into the circulation. Since different sub-clones of a tumor contribute to the circulating tumor DNA, circulating tumor variants may provide more than what we can generate from a typical tissue assay. Furthermore, sub-clones of the original tumor cells evolve over time thus changing the mutation profile detectable in the patient's blood. The present invention is a novel method as defined by the claims utilizing ecological diversity indices to analyze richness and abundance of the population of tumor cells in a patient in order to deliver an actionable result to the patient's physician.

Ecological diversity indices have previously been applied to tumors. Maley et al. (Genetic clonal diversity predicts progression to esophageal adenocarcinoma. Nat. Genet. 38, 468-473 (2006)), demonstrated the application of Shannon diversity in esophageal adenocarcinoma using multiple tissue biopsies, cell sorting, and FISH probes to TP53 and centromere of chromosome 17 to measure genetically distinct clones per sample. It was found that patients in top quartile in Shannon diversity index had increased probability of developing esophageal adenocarcinoma from a premalignant condition known as Barrett's esophagus. Almendro V, et al. (Inference of tumor evolution during chemotherapy by computation modeling and in situ analysis of genetic and phenotypic cellular diversity. Cell Reports. 6, 514-527 (2014)) used a similar approach to characterize diversity in breast cancer, while Merlo LMF et al. (A comprehensive survey of clonal diversity measures in Barrett's esophagus as biomarkers of progression to esophageal adenocarcinoma. Cancer Prevention Research Nov;3 (11):1388-97(2010)) showed that Shannon and Simpson are similar in esophageal adenocarcinoma.

The present invention is a comprehensive approach applying the ecological diversity indices to mutations detected in a patient's ctDNA. In some embodiments, the indices are Shannon index, Simpson index or a combination thereof. In some embodiments, the mutations are assessed by next-generation sequencing data applied to patient's ctDNA. The assessment of ecological diversity yields prognosis and proposed methods of treatment for the patient.

In some embodiments, the invention uses a biomarker panel to identify somatic mutations and mutation burden in cancer-related genes by next-generation sequencing (NGS). The invention utilizes a blood or blood -derived sample from a patient. The sample can include any fraction of blood, *e.g.,* serum or plasma, which contains cell-free DNA including circulating tumor DNA (cfDNA or ctDNA). In some embodiments, the sample is taken serially at various times during treatment, *e.g.,* before and after surgery or before, after and during a chemotherapy regimen. In some embodiments, a tumor sample such as a solid tumor sample is used for comparison with the blood sample. The solid tissue or blood sample can be collected by a suitable means that preserves the DNA therein, including formalin-fix paraffin-embedding (FFPE), fresh frozen tissue or blood tissue collected in a preservative medium.

The invention utilizes a biomarker panel as defined by Table 1. The use of a panel that contains a small portion of the genome (e.g., 1 megabase, 200 kilobases, 100 kilobases or less) is an improvement in efficiency over the existing methods such as whole genome sequencing (WGS) and whole exome sequencing (WES). The mutations assessed in the panel may include single-nucleotide variations (SNVs), deletions and insertions (in-dels) that correspond to on-sense missense and frame-shift mutations if they occur in the coding regions of genes. Other types of mutations include gene fusions and translocations. The selection, size and content of such panels has been described *e.g.,* in U.S. Patent applications Ser. No. 14/774,518 and International app. No. PCT/US2015/049838 *"Identification and Use of Circulating Tumor Markers."* The invention includes determining the sequence of at least a portion of the biomarkers listed in Table 1. In some embodiments, the entire sequence of a gene is determined. In other embodiments, the entire coding sequence of a gene is determined. In other embodiments, only the sequence of a portion of the gene known to undergo mutagenesis in cancer is determined. In yet other embodiments, the biomarker is not associated with a coding sequence but is associated with a regulatory sequence or a sequence of unknown function known to be mutated in human tumors.

In the context of the present invention, the sequence of a biomarker can be determined via any suitable method known in the art. The suitable method would have sufficient accuracy, *e.g.,* sensitivity and specificity to detect rare sequences with a low rate of errors. In some embodiments, the sequencing method includes an error correction step, such as use of molecular barcodes, error stereotyping and other chemical or computation methods of error suppression as described *e.g.,* in see the patent applications "Identification and Use of Circulating Tumor Markers", *supra.* The sequencing method may include a massively parallel sequencing method, including an array based sequencing (Illumina, San Diego, Cal.), an emulsion-based sequencing (ThermoFisher, Waltham, Mass.) an optical measurement based sequencing (Pacific BioSciences, Menlo Park, Cal.) or a nanopore-based sequencing (Roche Sequencing Solutions, Santa Clara, Cal.) or Oxford Nanopore (Oxford, UK), or any other single-molecule based sequencing method available.

In some embodiments, the invention utilizes a biomarker panel, such as AVENIO® ctDNA Analysis Kit (Roche Sequencing Solutions, Inc., Pleasanton, Cal.) that is capable of analyzing the tissue and blood of patients to identify and quantify tumor specific mutations in the samples. The composition of the biomarker panel is the AVENIO® ctDNA Analysis Kit surveillance panel shown in Table 1.

*Table 1. Composition of the surveillance biomarker panel*

| ABCC5 | CSMD1 | FAT1 | HTR1E | MAP7D3 | PIK3CA | SV2A |
|---|---|---|---|---|---|---|
| ABCG2 | CSMD3 | FBN2 | HTR2C | MKRN3 | PIK3CG | T |
| ACTN2 | CTNNB1 | FBXL7 | IFI16 | MMP16 | PKHD1L1 | THSD7A |
| ADAMTS1 2 | CTNND2 | FBXW7 | IL7R | MTX1 | POLE | TIAM1 |
| ADAMTS1 6 | CYBB | FCRL5 | INSL3 | MYH7 | POM121L12 | TMEM200 A |
| ARFGEF1 | DCAF12L 1 | FOXG1 | ITGA10 | MYT1L | PREX1 | TNFRSF21 |
| ASTN1 | DCAF12L 2 | FRYL | ITSN1 | NAV3 | PTPLA | TNN |
| ASTN2 | DCAF4L2 | GBA3 | KCNA5 | NEUROD 4 | RALYL | TNR |
| AVPR1A | DCLK1 | GBP7 | KCNB2 | NFE2L2 | RFX5 | TRHDE |
| BCHE | DCSTAMP | GJA8 | KCNC2 | NLGN4X | RIN3 | TRIM58 |
| BPIFB4 | DDI1 | GPR13 9 | KCNJ3 | NLRP3 | RNASE3 | TRPS1 |
| C6 | DLGAP2 | GRIA2 | KCTD8 | NMUR1 | ROBO2 | UGT3A2 |
| C6orf118 | DMD | GRIK3 | KEAP1 | NOL4 | SEMA5B | USH2A |
| CA10 | DNTTIP1 | GRIN2B | KIAA121 1 | NPAP1 | SLC18A3 | USP29 |
| CACNA1E | DOCK3 | GRIN3B | KIF17 | NROB1 | SLC39A12 | VPS13B |
| CDH12 | DSC3 | GRM1 | KIF19 | NRXN1 | SLC6A5 | WBSCR17 |
| CDH18 | DSCAM | GRM5 | KLHL31 | NXPH4 | SLC8A1 | WIPF1 |
| CDH8 | EGFLAM | GRM8 | KPRP | NYAP2 | SLITRK1 | WSCD2 |
| CDH9 | EPHA5 | GSX1 | LPPR4 | OPRD1 | SLITRK4 | ZC3H12A |

(continued)

| CDKN2A | EPHA6 | HCN1 | LRFN5 | P2RY10 | SLITRK5 | ZFPM2 |
|--------|-------|------|-------|--------|---------|-------|
| CHRM2 | EYS | HCRTR 2 | LRP1B | PAX6 | SLPI | ZIC1 |
| CNTN5 | FAM135B | HEBP1 | LRRC7 | PCDH15 | SMAD4 | ZIC4 |
| CNTNAP2 | FAM151A | HECW1 | LRRTM1 | PDYN | SOX9 | ZNF521 |
| CPXCR1 | FAM5B | HS3ST4 | LRRTM4 | PDZRN3 | SPTA1 | ZSCAN1 |
| CPZ | FAM5C | HS3ST5 | LTBP4 | PGK2 | ST6GALNAC 3 | KIT |
| CRMP1 | FAM71B | HTR1A | MAP2 | PHACTR1 | STK11 | NRAS |
| APC | KRAS | ALK | PDGFRA | MET | BRAF | RET |
| BRCA1 | BRCA2 | TP53 | DPYD | EGFR | ERBB2 | UGT1A1 |

In the invention, the panel of 197 genes listed in Table 1 is used.

In some embodiments, the step of determining the sequence of a biomarker comprises a target enrichment step. The enrichment may be by capturing the target sequences via one or more targets-specific probes. The nucleic acids in the sample may be denatured and contacted with single-stranded target-specific probes. The probes may comprise a ligand for an affinity capture moiety so that after hybridization complexes are formed, they are captured by providing the affinity capture moiety. In some embodiments, the affinity capture moiety is avidin or streptavidin and the ligand is biotin. In some embodiments, the moiety is bound to solid support. As described in further detail below, the solid support may comprise superparamagnetic spherical polymer particles such as DYNABEADS™ magnetic beads or magnetic glass particles.

In some embodiments, the step of determining the sequence of a biomarker further comprises an adaptor ligation step wherein adaptor molecules are ligated to the target nucleic acid. The ligation can be a blunt-end ligation or a more efficient cohesive-end ligation. The target nucleic acid may be rendered blunt-ended by "end repair" comprising strand-filling, *i.e.,* extending a 3'-terminus by a DNA polymerase to eliminate a 5'-overhang. In some embodiments, the blunt-ended nucleic acids may be rendered cohesive by addition of a single nucleotide to the 3'-end of the adaptor and a single complementary nucleotide to the 3'-ends of the target nucleic acid, *e.g.,* by a DNA polymerase or a terminal transferase. In yet other embodiments, the adaptors and the target nucleic acid may acquire cohesive ends (overhangs) by digestion with restriction endonucleases. The restriction enzyme recognition site may be inherent or engineered into the sequences. In some embodiments, other enzymatic steps may be required to accomplish the ligation. In some embodiments, a polynucleotide kinase may be used to add 5'-phosphates to the target nucleic acid molecules and adaptor molecules. In some embodiments, the adaptor molecules are *in vitro* synthesized artificial sequences. In other embodiments, the adaptor molecules are *in vitro* synthesized naturally-occurring sequences. In yet other embodiments, the adaptor molecules are isolated naturally occurring molecules.

In some embodiments, the step of determining the sequence of a biomarker further comprises a step of amplifying the target nucleic acid. The amplification may be by exponential polymerase chain reaction (PCR), linear amplification of only one strand or any other method that utilizes oligonucleotide primers. Various PCR conditions are described in PCR Strategies (M. A. Innis, D. H. Gelfand, and J. J. Sninsky eds., 1995, Academic Press, San Diego, CA) at Chapter 14; PCR Protocols : A Guide to Methods and Applications (M. A. Innis, D. H. Gelfand, J. J. Sninsky, and T. J. White eds., Academic Press, NY, 1990). The amplification step may take place before or after adaptor ligation. Accordingly, amplification utilizes a universal primer binding site introduced into the target sequence by e.g., adaptor ligation. In other embodiments, a gene-specific (target-specific) primer or primer pair is used prior to adaptor ligation and amplified target nucleic acids are ligated to the adaptors as described herein.

In some embodiments, the invention comprises introduction of barcodes into the target nucleic acids. Sequencing individual molecules typically requires molecular barcodes such as described *e.g.,* in U.S. Patent Nos. 7,393,665, 8,168,385, 8,481,292, 8,685,678, and 8,722,368. A unique molecular barcode is a short artificial sequence added to each molecule in a sample such as a patient's sample typically during the earliest steps of *in vitro* manipulations. The barcode marks the molecule and its progeny. The unique molecular barcode (UID) has multiple uses. Barcodes allow tracking each individual nucleic acid molecule in the sample to assess, *e.g.,* the presence and amount of circulating tumor DNA (ctDNA) molecules in a patient's blood in order to detect and monitor cancer without a biopsy. *See* U.S. patent application 14/774,518. Unique molecular barcodes can also be used for sequencing error correction. The entire progeny of a single target molecule is marked with the same barcode and forms a barcoded family. A variation in the sequence not shared by all members of the barcoded family is discarded as an artifact and not a true mutation. Barcodes can also be used for positional deduplication and target quantification, as the entire family represents a single molecule in the original sample. *See Id.*

In some embodiments, adaptors comprise one or more barcodes. In other embodiments, amplification primers (e.g., those used in amplification prior to adaptor ligation) comprise barcodes in the 5'-portion of the primer. A barcode can be a multiplex sample ID (MID) used to identify the source of the sample where samples are mixed (multiplexed). The barcode may also serve as a unique molecular ID (UID) used to identify each original molecule and its progeny. The barcode may also be a combination of a UID and an MID. In some embodiments, a single barcode is used as both UID and MID. In some embodiments, each barcode comprises a predefined sequence. In other embodiments, the barcode comprises a random sequence. Barcodes can be 1-20 nucleotides long.

In some embodiments, the step of determining the sequence of a biomarker further comprises a step of sequence analysis. The step comprises sequence aligning, error correction and determining sequence variations (mutations). In some embodiments, aligning is used to determine a consensus sequence from a plurality of sequences, *e.g.,* a plurality having the same barcodes (UID). In some embodiments barcodes (UIDs) are used to determine a consensus from a plurality of sequences all having an identical barcode (UID). In other embodiments, barcodes (UIDs) are used to eliminate artifacts, *i.e.,* variations existing in some but not all sequences having an identical barcode (UID). Such artifacts resulting from PCR errors or sequencing errors can be eliminated.

In some embodiments, the number of each sequence in the sample can be quantified by quantifying relative numbers of sequences with each barcode (UID) in the sample. Each UID represents a single molecule in the original sample and counting different UIDs associated with each sequence variant can determine the fraction of each sequence in the original sample. A person skilled in the art will be able to determine the number of sequence reads necessary to determine a consensus sequence. In some embodiments, the relevant number is reads per UID ("sequence depth") necessary for an accurate quantitative result. In some embodiments, the desired depth is 5-50 reads per UID.

The invention comprises a step of determining tumor variant diversity in a patient's sample by determining a diversity index, i.e., a quantitative measure that reflects how many different species there are in a dataset, and simultaneously takes into account how evenly the basic entities (such as individual mutant sequences) are distributed among those species. See Magurran, A.E., Measuring Biological Diversity, 2003 Wiley-Blackwell.

In some embodiments, the diversity index is a Shannon diversity index expressed as $(\sum_i p_i \ln(p_i))$, where $p_i$ is the proportion of species i in the population. A species is a variant of a sequence present in the patient's sample. Since proportion of all species in sample should add up to 1 (i.e., $\sum_i p_i = 1$), each $p_i$ is normalized by the sum of all $p_i$ for each sample. The number of species, i.e., variant (mutant) sequences is assessed to determine $p_i$. In some embodiments, the number of species is assessed as VARDEPTH, according to Formula I. In other embodiments, the number of species is assessed as another quantitative measurement, such as duplex depth (i.e., measured duplex molecules having the variant) or allele frequency.

In some embodiments, $p_i$, the proportion of species i in the population is calculated according to Formula I.

## *Formula I*

$$p_i = VARDEPTH_i / SVD$$

VARDEPTH = the deduplicated variant depth, or molecular depth, i.e., the number of unique molecules containing the variant (mutation) detected in the sequencing run;

SVD = $\sum_i VARDEPTH_i$ to represent the total of all molecules with detected variants (mutations)

Accordingly, the Shannon diversity index is expressed as Formula II.

## *Formula II*

$$Shannon = -\sum_i (VARDEPTH_i / SVD) \ln (VARDEPTH_i / SVD)$$

In some embodiments, the diversity index is the Simpson diversity index expressed as $\sum_i p_i^2$ (Formula III). In some embodiments, the diversity index is the Inverse Simpson index expressed as $1/\sum_i p_i^2$ (Formula IV). In some embodiments, the diversity index is the Gini-Simpson index expressed as $1 - \sum_i p_i^2$ (Formula V). In all embodiments, $p_i$ is the proportion of species *i* in the population calculated as set forth above (e.g., according to Formula I or using a quantitative measurement alternative to VARDEPTH utilized in Formula I instead of VARDEPTH).

The tumor variant diversity index determined according to the instant invention is assessed as being in the same quantile as the tumor variant diversity index of patients in a relevant population who have had a good outcome or is in the same quantile as the tumor variant diversity of patients in the relevant population who have had a poor outcome. In some embodiments, the tumor variant diversity is assessed at the population level wherein the relevant population consists of cancer patients diagnosed with the same type of cancer. The quantile may be a quartile, a tertile or a median. In some embodiments, the quantile is a tertile and the tumor variant diversity is defined as low if it falls under the first tertile in the population and the tumor variant diversity is defined as high if it falls at or above the first tertile in the population.

The invention includes a step of assessing the status of a cancer in a patient using the tumor variant diversity index obtained from the patient's ctDNA. In some embodiments, the assessing includes identifying the patient as likely or not likely to respond to anti-cancer therapy by determining prognosis of a patient by the method as claimed. In some embodiments, response to therapy is assessed as predicted overall survival (OS) after completion of the therapy. In some embodiments, the therapy is first-line chemotherapy or chemoradiation therapy. The assessing is based on the method as claimed. In some embodiments, the assessment step including the step of determining patient's prognosis can be done using the population data as set forth above.

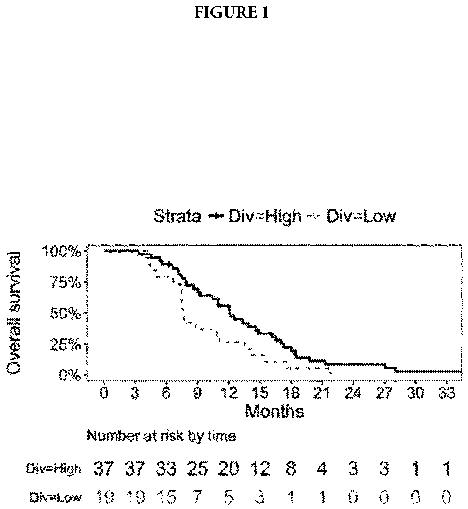

The examples set forth below illustrate the use of tumor diversity index in different populations, *i.e.,* patients diagnosed with different types of cancer. In the first example, as shown on Figure 1, Stage IV small cell lung cancer (SCLC) patients with lower Shannon diversity index had a poor prognosis compared to patients with a higher Shannon diversity index. The index was assessed at baseline (before chemotherapy). Patients with variant duplex depth in the first tertile ($\leq$ 1.17) had shorter overall survival (hazard ratio = 1.8; 95% CI 1-3.3; log-rank p = 0.034; median survival difference = 4.5 months).

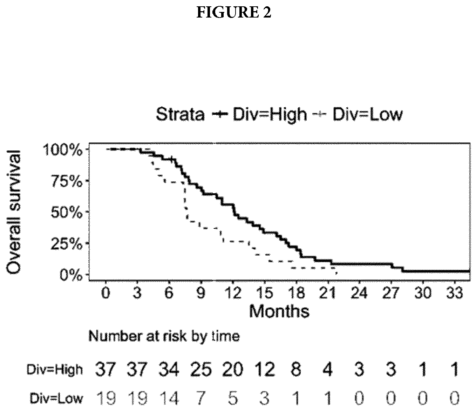

In another example, as shown in Figure 2, stage IV small cell lung cancer (SCLC) patients with lower Gini-Simpson diversity index had a poor prognosis compared to patients with a higher Gini-Simpson diversity index. The index was assessed at baseline (before chemotherapy). Patients with variant duplex depth in the first tertile ($\leq$ 0.64) had shorter overall survival (hazard ratio = 1.8; 95% CI 1-3.3; log-rank p = 0.033; median survival difference = 4.5 months). Gini-Simpson and inverse Simpson values gave the same survival analyses results.

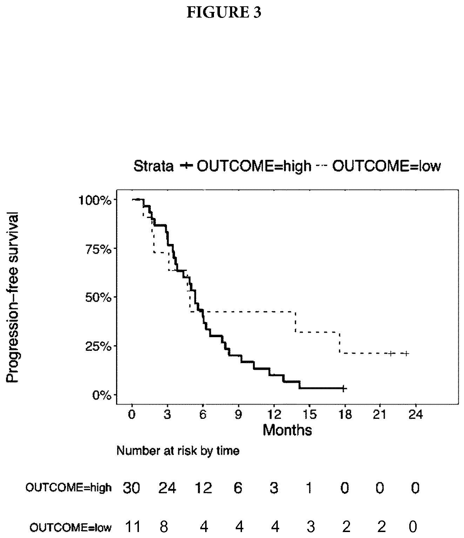

In an example with a different population, stage IV lung adenocarcinoma (non-small cell lung cancer, NSCLC) patients with lower Gini-Simpson diversity index had a better prognosis compared to patients with a higher Gini-Simpson diversity index (Figure 3). The index was assessed at baseline (before chemotherapy). Patients with variant duplex depth in the first quartile ($\leq$ 0.65) had longer progression-free survival after 6 months on chemotherapy treatment. Gini-Simpson and inverse Simpson values gave the same survival analyses results.

One aspect of the method as claimed may be carried out with the help of a system for detecting tumor variant diversity in a patient. The system comprises a processor and a non-transitory computer readable medium coupled to the processor, the medium comprising code executable by the processor for performing a method comprising the steps of analyzing sequencing data on biomarkers from Table 1, performing sequence comparison and mutation detection, error correction, determining tumor variant diversity according to Formula II, III, IV or V and whether the tumor variant diversity in the sample falls above or below a predetermined threshold, *e.g.,* a population-based threshold.

In some embodiments, the computer readable medium, which may include one or more storages devices, comprises a database including a listing of available therapies depending on tumor variant diversity in the patient. The computer readable medium further comprises a program code having instructions to generate a report listing suitable therapies.

The system may comprise various functional aspects such a server including a processor for processing digital data, a memory coupled to the processor for storing digital data, an input digitizer coupled to the processor for inputting digital data, program code stored in the memory and accessible by the processor, a display device coupled to the processor and memory for displaying information derived from digital data, data networking, and one or more informational databases. The databases may include patient data, patient sample data, clinical data including prior treatment data, a list of therapies and therapeutic agents, patient tracking data and the like.

## EXAMPLES

*Example 1. Assessing tum or variant diversity in Small Cell Lung Cancer (SCLC) patients*

In this example, pre-treatment plasma samples were obtained from 56 subjects with Stage IV small cell lung cancer (SCLC). The subjects have been previously treated with first-line chemotherapy or chemoradiation therapy. Plasma samples were analyzed with the AVENIO® ctDNA Surveillance Kit (Roche Sequencing Solutions, Pleasanton, Cal.), a targeted next-generation sequencing panel of 198 kilobases (Table 1). The samples were processed according to manufacturer's recommendations. The sequencing data was analyzed according to the manufacturer's recommendations to determine variants in the sequence reads.

Shannon diversity index (Formula I) and Simpson diversity index (Formula II) were applied to the variants data by considering each somatic variant as a species and the number of detected duplex molecules with that mutation as the abundance of that species. Samples were ranked as low tumor heterogeneity if their plasma variant diversity score was below the first tertile of the cohort.

Results demonstrate that stage IV SCLC subjects with low tumor variant diversity evaluated as Shannon diversity index had shorter overall survival (hazard ratio = 1.8; 95% CI 1-3.3; log-rank p = 0.034; median survival difference = 4.5 months; Figure 1). Similarly, subjects with low tumor variant diversity evaluated as Gini-Simpson index or inverse Simpson diversity index had shorter overall survival (hazard ratio = 1.8; 95% CI 1-3.3; log-rank p = 0.033; median survival difference = 4.5 months; Figure 2).

*Example 2. Assessing tumor variant diversity in Non-Small Cell Lung Cancer (NSCLC) patients*

We also evaluated the Simpson diversity indices on pre-treatment plasma samples from a prospective, observational study of 41 Stage IV lung adenocarcinoma (Non-Small Cell Lung Cancer (NSCLC)). The subjects have been previously treated with first-line chemotherapy or chemoradiation therapies. Plasma samples were analyzed with the AVENIO® ctDNA Surveillance Kit (Roche Sequencing Solutions, Pleasanton, Cal.), a targeted next-generation sequencing panel of 198 kilobases (Table 1). The samples were processed according to manufacturer's recommendations. The sequencing data was analyzed according to the manufacturer's recommendations to determine variants in the sequence reads.

Shannon diversity index (Formula I) and Simpson diversity index (Formula II) were applied to the variants data. Samples were classified as low tumor variant diversity if their plasma variant diversity score was below the first tertile of the training cohort. Stage IV adenocarcinoma subjects with low tumor variant diversity assessed as the Gini-Simpson index or inverse Simpson diversity index had longer progression-free survival after 6 months on chemotherapy treatment (Figure 3).

## Claims

1. A method of identifying a prognosis for a cancer patient comprising the steps of:

   (a) isolating nucleic acids from a cell-free blood sample obtained from the patient;
   (b) determining in the samples the sequence of at least a portion of each of the biomarkers listed in Table 1; wherein said portion may include single-nucleotide variations (SNVs), deletions and insertions (in-dels) that correspond to nonsense missense and frame-shift mutations if they occur in the coding regions of genes, gene fusions or translocations.
   (c) determining a tumor variant diversity index in the patient;
   (d) identifying the patient as having a good prognosis if the tumor variant diversity index is in the same quantile as the tumor variant diversity index of patients in a relevant population who have had a good outcome; or
   (e) identifying the patient as having a poor prognosis if the tumor variant diversity index is in the same quantile as the tumor variant diversity index of patients in the relevant population who have had a poor outcome, wherein the cancer is selected from among non-small cell lung cancer (NSCLC), small cell lung cancer (SCLC),

   wherein the tumor variant diversity index is selected from Shannon diversity index, Simpson diversity index, Inverse Simpson diversity index and Gini-Simpson diversity index, and
   wherein the prognosis is overall survival (OS).

2. The method of claim 1, wherein the diversity index is determined using the proportion of species in a population determined according to Formula I:

$$p_i = VARDEPTH_i \,/\, SVD.$$

3. The method of claim 1, wherein the Shannon diversity index is determined according to Formula II:

$$Shannon = - \sum i\ (VARDEPTHi\ /SVD)\ \ln\ (VARDEPTHi\ /SVD)$$

   or

$$III:\ \ \sum i\ pi2$$

4. The method of claim 1, wherein the Inverse Simpson diversity index is determined according to Formula IV: $1/\Sigma ipi2$

5. The method of claim 1, wherein the Gini-Simpson diversity index is determined according to Formula V: $1 - \Sigma_i p_i^{\,2}$

6. The method of claim 1, wherein the relevant population is the population of patients having the same type of cancer.

7. The method of claim 1, wherein determining the sequence comprises a step of target enrichment.

8. The method of claim 1, wherein determining the sequence comprises a step of adaptor ligation.

9. The method of claim 1, wherein determining the sequence utilizes molecular barcodes.

10. The method of claim 1, wherein determining the sequence comprises a step of sequence alignment.

11. The method of claim 1, wherein determining the sequence comprises a step of error correction.


**Patentansprüche**

1. Verfahren zum Identifizieren einer Prognose für einen Krebspatienten, umfassend die folgenden Schritte:

(a) Isolieren von Nukleinsäuren aus einer zellfreien Blutprobe, die von dem Patienten erhalten wurde;
(b) Bestimmen der Sequenz mindestens eines Teils jedes der in Tabelle 1 aufgeführten Biomarker in den Proben; wobei der Teil Einzelnukleotidvariationen (SNVs), Deletionen und Insertionen (In-Dels) umfassen kann, die Nonsense- Missense- und Frameshift-Mutationen entsprechen, wenn sie in den kodierenden Bereichen von Genen, Genfusionen oder Translokationen auftreten.
(c) Bestimmen eines Tumorvarianten-Diversitätsindex bei dem Patienten;
(d) Identifizieren des Patienten als Patient mit guter Prognose, wenn der Tumorvarianten-Diversitätsindex im gleichen Quantil liegt wie der Tumorvarianten-Diversitätsindex von Patienten in einer relevanten Population, die einen guten Ausgang hatten; oder
(e) Identifizieren des Patienten als Patient mit schlechter Prognose, wenn der Tumorvarianten-Diversitätsindex im gleichen Quantil liegt wie der Tumorvarianten-Diversitätsindex von Patienten in der relevanten Population, die einen schlechten Ausgang hatten, wobei der Krebs ausgewählt ist aus nicht-kleinzelligem Lungenkrebs (NSCLC), kleinzelligem Lungenkrebs (SCLC), wobei der Tumorvarianten-Diversitätsindex ausgewählt ist aus Shannon-Diversitätsindex, Simpson-Diversitätsindex, Inverse-Simpson-Diversitätsindex und Gini-Simpson-Diversitätsindex, und wobei die Prognose die Gesamtüberlebenszeit (OS) ist.

2. Verfahren nach Anspruch 1, wobei der Diversitätsindex unter Verwendung des Anteils von Spezies in einer Population bestimmt wird, der gemäß Formel I bestimmt wird:

$$p_i = VARDEPTH_i \; / \; SVD.$$

3. Verfahren nach Anspruch 1, wobei der Shannon-Diversitätsindex bestimmt wird gemäß Formel II:

$$Shannon = - \sum i \; (VARDEPTHi \; /SVD) \; \ln \; (VARDEPTHi \; /SVD)$$

oder

$$III: \sum i \; pi2$$

4. Verfahren nach Anspruch 1, wobei der Inverse-Simpson-Diversitätsindex gemäß Formel IV bestimmt wird: $1/\sum ipi2$

5. Verfahren nach Anspruch 1, wobei der Gini-Simpson-Diversitätsindex gemäß Formel V bestimmt wird: $1 - \sum_i p_i^2$

6. Verfahren nach Anspruch 1, wobei die relevante Population die Population von Patienten mit dem gleichen Krebstyp ist.

7. Verfahren nach Anspruch 1, wobei das Bestimmen der Sequenz einen Schritt der Zielanreicherung umfasst.

8. Verfahren nach Anspruch 1, wobei das Bestimmen der Sequenz einen Schritt der Adapterligation umfasst.

**9.** Verfahren nach Anspruch 1, wobei das Bestimmen der Sequenz molekulare Barcodes verwendet.

**10.** Verfahren nach Anspruch 1, wobei das Bestimmen der Sequenz einen Schritt des Sequenzabgleichs umfasst.

**11.** Verfahren nach Anspruch 1, wobei das Bestimmen der Sequenz einen Schritt der Fehlerkorrektur umfasst.

**Revendications**

**1.** Procédé d'identification d'un pronostic pour un patient atteint de cancer comprenant les étapes de :

(a) isolement d'acides nucléiques d'un échantillon de sang exempt de cellules obtenu auprès du patient ;
(b) détermination dans les échantillons de la séquence d'au moins une partie de chacun des biomarqueurs répertoriés dans le tableau 1 ; ladite partie pouvant comprendre des variations mononucléotidiques (SNV), des délétions et des insertions (in-dels) qui correspondent à des mutations non-sens, faux sens et à décalage du cadre de lecture si elles se produisent dans les régions codantes de gènes, des fusions de gènes ou des translocations ;
(c) détermination d'un indice de diversité de variants de tumeur chez le patient ;
(d) identification du patient comme ayant un pronostic favorable si l'indice de diversité de variants de tumeur est dans le même quantile que l'indice de diversité de variants de tumeur de patients dans une population pertinente ayant présenté une issue favorable ; ou
(e) identification du patient comme ayant un pronostic défavorable si l'indice de diversité de variants de tumeur est dans le même quantile que l'indice de diversité de variants de tumeur de patients dans la population pertinente ayant présenté une issue défavorable, dans lequel le cancer est choisi parmi le cancer du poumon non à petites cellules (CPNPC), le cancer du poumon à petites cellules (CPPC),
dans lequel l'indice de diversité de variants de tumeur est choisi parmi l'indice de diversité de Shannon, l'indice de diversité de Simpson, l'indice de diversité inverse de Simpson et l'indice de diversité de Gini-Simpson, et
dans lequel le pronostic est la survie globale (SG).

**2.** Procédé selon la revendication 1, dans lequel l'indice de diversité est déterminé en utilisant la proportion d'espèces dans une population déterminée selon la formule I :

$$p_i = VARDEPTH_i \, / \, SVD.$$

**3.** Procédé selon la revendication 1, dans lequel l'indice de diversité de Shannon est déterminé selon la formule II :

$$Shannon = - \sum i \, (VARDEPTHi \, /SVD) \, \ln \, (VARDEPTHi \, /SVD)$$

ou

$$III : \sum i \, pi2.$$

**4.** Procédé selon la revendication 1, dans lequel l'indice de diversité inverse de Simpson est déterminé selon la formule IV : $1/\Sigma ipi2$.

**5.** Procédé selon la revendication 1, dans lequel l'indice de diversité de Gini-Simpson est déterminé selon la formule V : $1 - \Sigma_i p_i^2$.

**6.** Procédé selon la revendication 1, dans lequel la population pertinente est la population de patients ayant le même type de cancer.

**7.** Procédé selon la revendication 1, dans lequel la détermination de la séquence comprend une étape d'enrichissement de cible.

**8.** Procédé selon la revendication 1, dans lequel la détermination de la séquence comprend une étape de ligation d'adaptateur.

**9.** Procédé selon la revendication 1, dans lequel la détermination de la séquence utilise des codes-barres moléculaires.

**10.** Procédé selon la revendication 1, dans lequel la détermination de la séquence comprend une étape d'alignement de séquences.

**11.** Procédé selon la revendication 1, dans lequel la détermination de la séquence comprend une étape de correction d'erreurs.

**FIGURE 1**

FIGURE 2

## FIGURE 3

Strata + OUTCOME=high -- OUTCOME=low

Number at risk by time

|  | 0 | 3 | 6 | 9 | 12 | 15 | 18 | 21 | 24 |
|---|---|---|---|---|---|---|---|---|---|
| OUTCOME=high | 30 | 24 | 12 | 6 | 3 | 1 | 0 | 0 | 0 |
| OUTCOME=low | 11 | 8 | 4 | 4 | 4 | 3 | 2 | 2 | 0 |

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 774518
- US 2015049838 W
- US 7393665 B
- US 8168385 B
- US 8481292 B
- US 8685678 B
- US 8722368 B

**Non-patent literature cited in the description**

- **ABBOSH et al.** *Nature*, 2017, vol. 545 (7655), 446-451
- **CHUNG et al.** *Oncotarget*, 2017, vol. 8 (57), 97114-97126
- **MAGURRAN, A.E.** Measuring Biological Diversity. Wiley-Blackwell, 2003
- **MALEY et al.** Genetic clonal diversity predicts progression to esophageal adenocarcinoma.. *Nat. Genet.*, 2006, vol. 38, 468-473
- **ALMENDRO V et al.** Inference of tumor evolution during chemotherapy by computation modeling and in situ analysis of genetic and phenotypic cellular diversity.. *Cell Reports*, 2014, vol. 6, 514-527
- **MERLO LMF et al.** A comprehensive survey of clonal diversity measures in Barrett's esophagus as biomarkers of progression to esophageal adenocarcinoma. *Cancer Prevention Research*, November 2010, vol. 3 (11), 1388-97
- PCR Strategies. Academic Press, 1995
- PCR Protocols : A Guide to Methods and Applications. Academic Press, 1990